# EUROPEAN PATENT APPLICATION

(11) **EP 2 842 943 A1**
(43) Date of publication of application: **04.03.2015**
(21) Application number: 13306191.1
(22) Date of filing: 30.08.2013
(51) Int. Cl.: C07D 213/68, C09K 11/06

(54) **New molecules presenting white light emission properties**

(71) Applicant: Université de Strasbourg, 67000 Strasbourg (FR); Centre National de la Recherche Scientifique, 75016 Paris (FR); Sun Yat-Sen University, Guangzhou 510275 (CN)
(72) Inventor: Lehn, Jean Marie, 67000 Strasbourg (FR); Yang, Qing-Yuan, 67000 Strasbourg (FR)
(74) Representative: Blot, Philippe Robert Emile

(57) **Abstract**

The present invention relates to new molecules having white light emission properties, their method of preparation, and their applications, notably as white light emitting sources.

## Description

The present invention relates to new molecules presenting white light emission properties, their method of preparation and their applications, notably as white light emitting sources.

Emission of white light from a single molecule is an important chemical target because of the possibility of innovative applications to new classes of displays and light sources, such as low-cost, large-area flat-panel displays. Most of the white organic light-emitting devices (WOLEDs) reported so far relied on the use of a combination of several organic components that emit different colors of light (e.g. red/blue/green luminophores) to cover the visible range from 400 to 700 nm. These WOLEDs may degrade at different rates or have different requirements for device fabrication. Compared to the multi-emitting component WOLEDs, use of a single molecule could show many advantages, such as better stability, better reproducibility, and a simpler fabrication process. However, only a few molecules are known to show white-light emission. Therefore, the search for new molecules that can emit white light is of obvious interest and importance.

Until now, all of known white light emitting molecules have complicated structures and their preparation requires complex synthetic strategies. These materials are usually composed of covalently linked emitter components and utilize energy transfer from a wide bandgap donor component to a smaller band-gap acceptor to generate white light. The complexity of these molecules may hinder their market entry. So achieving white light emission by single molecules with simple structure is currently an exciting challenge in synthetic chemistry as well as in photochemistry and photophysics.

### Aims of the invention

The present invention aims to solve the above recited technical problems.

In particular, the invention aims to provide new white light emitting molecules.

The invention aims in particular to provide such compounds while avoiding the complex composition or complex structure of known white light emitting molecular systems.

The invention also aims to provide such compounds with good electroluminescent properties.

The invention aims to provide such compounds with good lifetime and quantum yield.

The invention also aims to provide a simple way to prepare white light emitting molecules.

### Description of the invention

The inventors have discovered new compounds (or molecules) and materials presenting white light emitting properties.

The compounds according to the invention have simple molecule structure and some of them can emit white light in the solid state.

The invention relates to compound of formula (I) 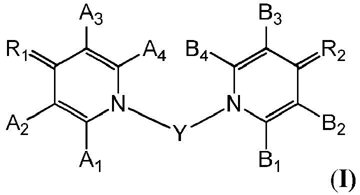 wherein Y represents a straight or branched alkyl chain having from 1 to 30 carbon atoms, preferably from 2 to 8 or from 9 to 20 carbon atoms, said alkyl chain optionally containing one or more unsaturations, and/or optionally containing one or more heteroatoms, and/or being optionally substituted, said alkyl chain being optionally interrupted by an aromatic group,
- R₁ and R₂ represent each independently an oxygen atom or 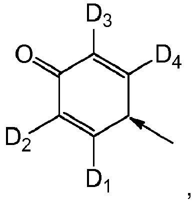
- ↗ indicating the point of attachment of the covalent double bond,
- A₁, A₂, A₃, A₄, B₁, B₂, B₃, B₄, D₁, D₂, D₃, and D₄ are identical or different, and represent each independently a hydrogen atom, a halogen atom (F, Cl, Br, or I), an alkyl, O-alkyl or aromatic group, A₁-A₂, A₃-A₄, B₁-B₂, B₃-B₄, D₁-D₂, and D₃-D₄ may each represent independently an aromatic ring.

Preferably, said alkyl chain or group is selected from the group consisting of methane, ethane, propane, butane, pentane, hexane, octane, nonane, decane, dodecane, tridecane, tetradecane, pentadecane, hexadecane, heptadecane, octadecane, nonadecane, eicosane, isopropane, tert-butyl, 2-ethylhexane, 2-methyl butane, 2-methylpentane, 1-methylpentane, 3-methylheptane, benzene, naphthalene, anthracene, perylene, diphenyl, bipyridine and terpyridine.

According to a preferred embodiment, said alkyl chain or group contains one or two heteroatoms, said heteroatom being independently selected from O, N, and S.

At least one carbon atom of said alkyl chain or group may be substituted by one of the following atoms or groups:
- F, Cl, Br, or I,
- C₁-C₃₀ alkyl, C₃-C₇ cycloalkyl, C₆-C₁₀ aryl, heteroaryl, aralkyl, heteroarylalkyl, wherein said alkyl or aryl is optionally substituted by 1 to 3 groups R²⁰, wherein R²⁰ is one of the following:
- OR²², NR²³R²⁴, NHOH, NO₂, CN, CF₃, C₁-C₆ alkyl, C₂-C₆ alcenyl, C₂-C₆ alcynyl, C₆-C₁₀ aryl, aralkyl, =O, C(=O)R²², CO₂R²², OC(=O)R²², C(=O)NR²³R²⁴, OC(=O)NR²³R²⁴, NR²¹C(=S)R²² or S(O)_{y}R²²;
- R²² is, at each occurrence, independently selected among H, C₁-C₃₀ alkyl, preferably C₅-C₂₀ alkyl, C₆-C₁₀ aryl and aralkyl ;
- R²³ and R²⁴ are, at each occurrence, independently selected among H, C₁-C₃₀ alkyl_{,} preferably C₅-C₂₀ alkyl, C₆-C₁₀ aryl, aralkyl, or R²³ and R²⁴ form together a heterocyclic group having from 3 to 7 atoms.

According to one embodiment, said alkyl chain Y is interrupted by an aromatic group and is represented by: 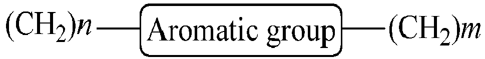 wherein *n* and *m* are integers and wherein 2 < n + m < 30.

According to a specific embodiment, said aromatic group is a phenyl group, optionally substituted.

According to a preferred embodiment, compounds of formula (I) are those wherein 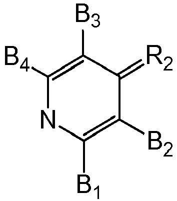 of formula (I) represents one of the following chemical groups: 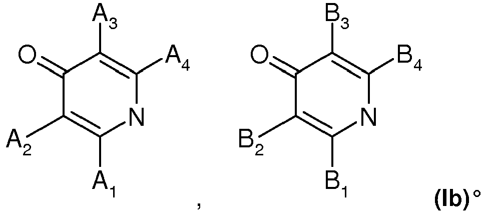 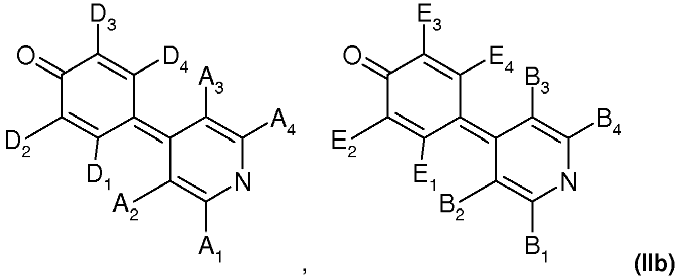 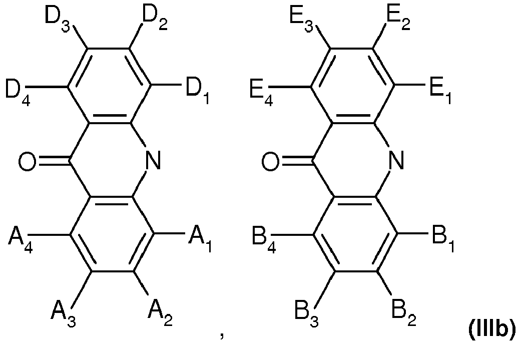 wherein A₁, A₂, A₃, A₄, B₁, B₂, B₃, B₄, D₁, D₂, D₃, and D₄ are defined according to the present invention, and E₁, E₂, E₃, E₄ are identical or different, and represent each independently a hydrogen atom, a halogen atom (F, Cl, Br, or I), an alkyl, O-alkyl or aromatic group, E₁-E₂, and E₃-E₄ may each represent independently an aromatic ring.

According to a more specific embodiment, compounds of the invention are represented by one of the following formulae:

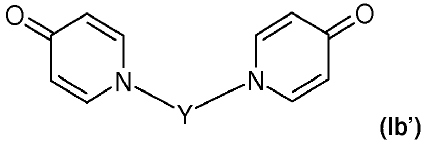

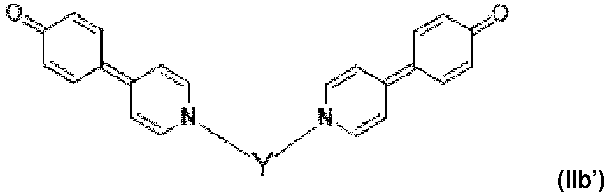

According to one embodiment, compounds of the invention are represented by the following formula: 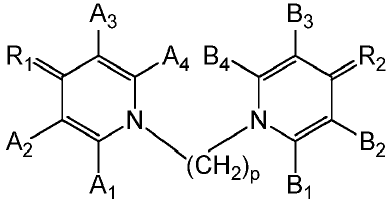 wherein *p* is an integer and wherein 1 < p < 30.

White light is composed of the three primary colors (red, green and blue) and radiations wavelengths cover the whole visible range from 400 to 700 nm.

According to the present invention, a molecule emitting white light emits radiations presenting a normalized intensity in arbitrary units (a.u.) higher than 0.1 essentially or totally over 400 to 700 nm, and preferably higher than 0.5 over radiations wavelengths ranging from 425 to 575 nm, preferably an intensity higher than 0.7 over a range of 100nm breadth. Preferred molecules are those emitting radiations wavelengths in the visible range, i.e. from 400 to 700 nm, said radiations having an intensity higher than 0.7, even 0.8, over a range of at least 150 nm breadth, more preferably of at least 200nm breadth.

According to the CIE 1931 XYZ color space, created by the Commission Internationale de l'éclairage (CIE) in 1931, the CIE chromaticity coordinates for pure white light are (0.33, 0.33).

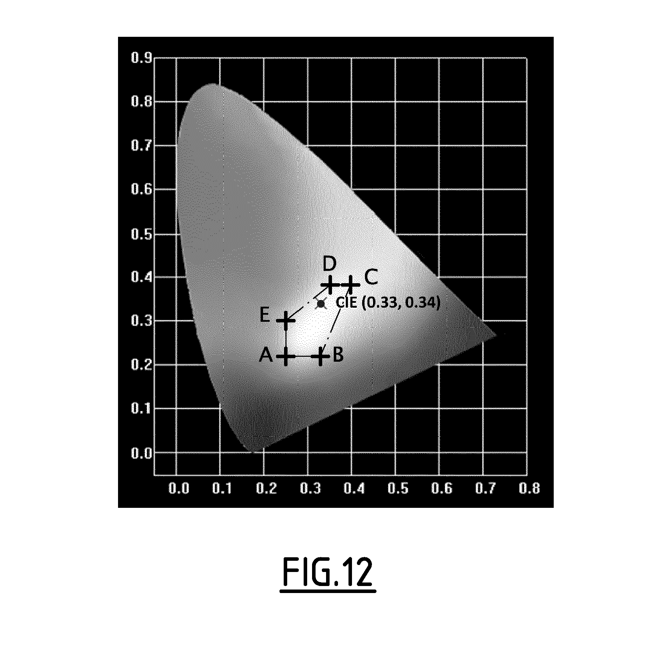

White light according to the present invention is more particularly defined by CIE coordinates in the (0.33±0.10, 0.33±0.10) range, and more preferably in the (0.33±0.07, 0.33±0.07) range. Compounds of the invention present preferably CIE chromaticity coordinates on the surface of a simple pentagon defined by points A (0.25, 0.22); B (0.33, 0.22); C (0.40, 0.38); D (0.35, 0.38); E (0.25, 0.30). A simple pentagon is notably a pentagon in which the boundary of the pentagon does not cross itself. Such a pentagon is illustrated in figure 12. Even more preferably, compounds of the invention present CIE coordinates close to or equal to those of pure white light (0.33±0.05, 0.33±0.05).

The invention also relates to the use of at least one compound of the invention, or of a solid phase of the invention, for emiting white light. The invention also relates to a method for emiting white light.

Typically, white light is emitted by the compounds of the invention when they are exposed to UV, laser, or electric sources.

The compounds according to the invention provide a good quantum yield, which is defined as the quotient of the number of photons emitted divided by the number of photons absorbed. The quantum yield represents the emission efficiency of a given compound.

A good quantum yield is considered to be over 18%, preferably over 20%.

Lifetime (τ) is defined as the average length of time for the molecules to decay from one state to another. When a molecule absorbs a photon of appropriate energy, a chain of photophysical events ensues, such as internal conversion, fluorescence, intersystem crossing, and phosphorescence, as shown in the Jablonski diagram. Each of the processes occurs with a certain probability, characterized by decay rate constants (*k*). Lifetime (τ) is reciprocally proportional to the rate of decay: τ = 1/*k*. The lifetime can be considered as a state function because it does not depend on excitation wavelength.

Electroluminescent is defined as an optical phenomenon and electrical phenomenon in which a material emits light in response to the passage of an electric current or to a strong electric field. During operation, a current of electrons flows through the emitting materials from cathode to anode. Electrostatic forces bring the electrons and the holes towards each other and they recombine forming an exciton, a bound state of the electron and hole. The decay of this excited state results in the release of energy. The emitting materials absorb the energy accompanied by emission of radiation as visible light. The colour of this light depends on the band gap between the HOMO and LUMO.

The lifetime of a molecule of the invention is in general of between 1 and 10 nanoseconds, more specifically from 2 to 3 nanoseconds.

The invention also relates to a solid state material comprising, or consisting of, at least one compound of the invention.

According to one embodiment, said solid state or phase material is a pulverulent material.

One or more compounds of the invention may be introduced as a solid powder in a polymeric material or in a porous solid, such as a zeolite or a metal organic framework (MOF). Accordingly the invention relates to a polymeric material or a solid state material comprising at least one compound of the invention.

According to a specific embodiment the polymeric material is a plastic material, especially a transparent plastic material. For example said plastic material is a plastic film, especially a transparent plastic film.

Compounds of the invention may also be combined with and introduced into solid state host materials, such as organic or inorganic porous frameworks or material, clays, zeolites, etc.

The invention also relates to a light emiting device comprising at least one compound of the invention, or a solid phase of the invention. More specificly, said device may be a white light meiting device.

According to a specific embodiment, the invention relates to one or more light sources comprising one or more compounds of the invention. According to one embodiment, the light source is a WOLED or a plurality of WOLEDs comprising one or more compounds of the invention.

The invention also relates to a method for producing white light wherein said method comprises exciting one or more compounds or materials of the invention with a source of energy to produce white light. In one embodiment the source of energy is a UV light source, a laser source, an electric source, or any combination thereof.

Compounds of the invention are very simple to prepare as they require only two main steps. For example, a method for preparing a compound according to formula (I) comprises the condensation of a chain with two molecules of 4-hydroxypyridine.

Thus the invention relates to a method for preparing a compound of the invention, wherein said method comprises the following steps : 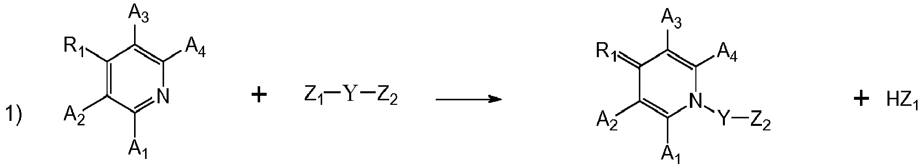 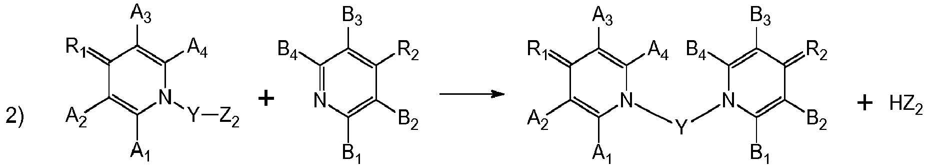 wherein Z₁ and Z₂ represent each independently leaving groups, preferably Z₁ and Z₂ represent each independently a halogen atom (preferably Br), a sulfonyl group, preferably a tosyl, a methanesulfonyl, a triflyl, a besyl, or a sulfonate group, or a sulphate ester group (-O-SO₃-R);

R₁, R₂, Y, A₁, A₂, A₃, A₄, B₁, B₂, B₃, and B₄ being defined according to the present invention.

For example, a method for preparing a compound according to formula (**Ib**) may comprise the following steps :

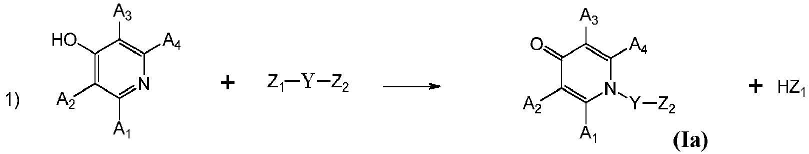

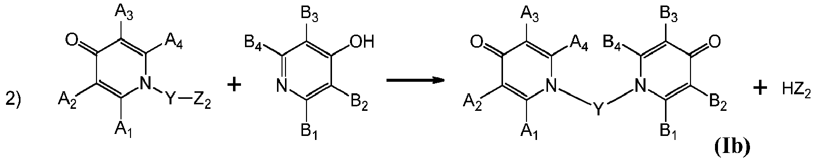

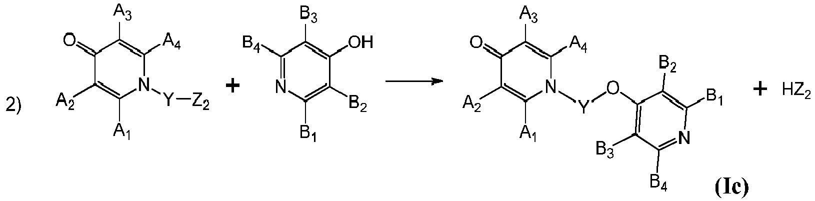

For example, a method for preparing a compound according to formula (**IIb**) may comprise the following steps : 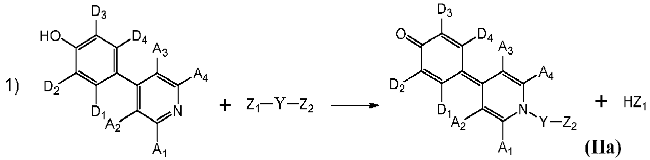 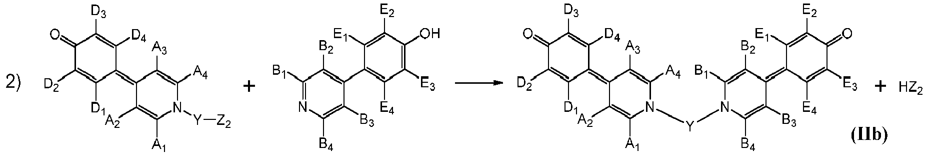 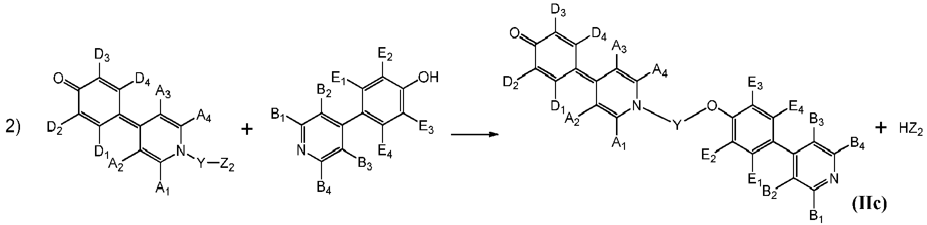 wherein A₁, A₂, A₃, A₄, B₁, B₂, B₃, B₄, D₁, D₂, D₃, D₄, E₁, E₂, E₃, E₄, Z₁ and Z₂ are defined in the present invention.

According to a preferred embodiment Z₁ and Z₂ are bromine atoms (Br). Preferably Z₁-Y-Z₂ is a dihalogenoalkyl, and more preferably a dibromoalkyl.

According to a particular embodiment, A₁=B₁, A₂=B₂, A₃=B₃, A₄=B₄ in formula **(Ib).**

According to a particular embodiment, A₁=B₁, A₂=B₂, A₃=B₃, A₄=B₄, D₁=E₁, D₂=E₂, D₃=E₃, D₄=E₄ in formula **(IIb).**

According to one embodiment step 1) and/or 2) are performed in the presence of a base, for example K₂CO₃, and an organic solvent.

The reaction can be performed under reflux.

According to a preferred embodiment the reaction is a one pot reaction wherein steps 1 and 2 are performed in the same reaction vessel.

After the reaction is complete, the organic solvent may be removed in order to collect the crude product of the reaction. More precisely the crude product generally contains: 1) the desired symmetrical compound **Ib** or **IIb**, where the central chain is connected to the nitrogen site of the two terminal heterocyclic groups; 2) the unsymmetrical compound **Ic** and **IIc**, where the central chain is connected to the nitrogen site of one terminal heterocyclic group and to the oxygen of the other one; 3) unreacted remaining 4-hydroxypyridine. This product is advantageously purified for instance by washing with cold water to remove the 4-hydroxypyridine followed by washing with acetone at room temperature to remove the unsymmetrical c isomer. After that, the desired symmetrical compound was recrystallized from acetonitrile. This procedure provides an essentially pure product. "Essentially pure" means a pure product with unavoidable impurities present because of the raw materials used and the reaction performed. It is typically > 99.2% pure.

### In the figures

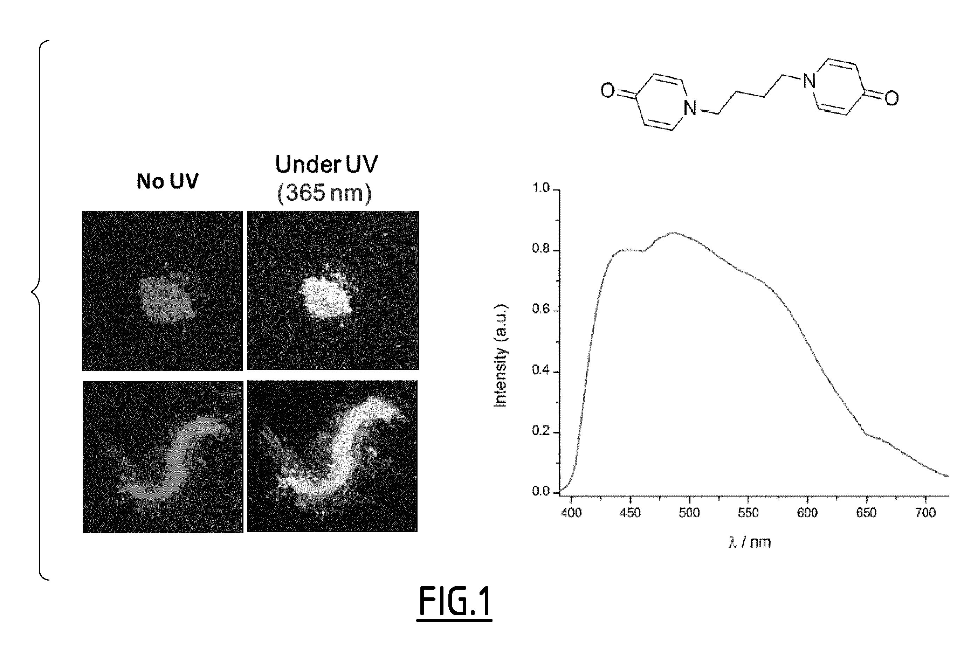

FIGURE 1. (Left) Photos of a powder of molecule **1** in absence of UV irradiation and under excitation at 365 nm. (Right) Solid state emission spectrum of **1** obtained as described, under excitation at 365 nm.

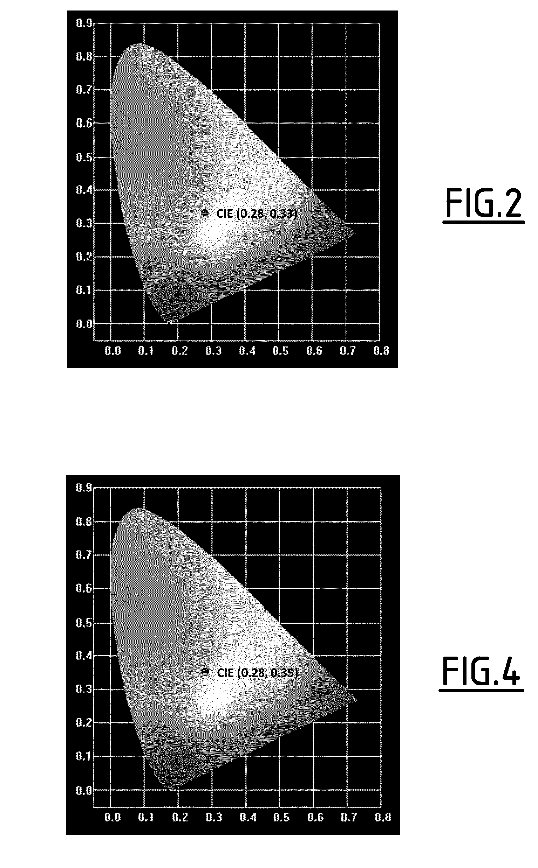

FIGURE 2. Molecule **1** emits white blue light in the solid state upon excitation at 365 nm with CIE coordinates are (0.28, 0.33).

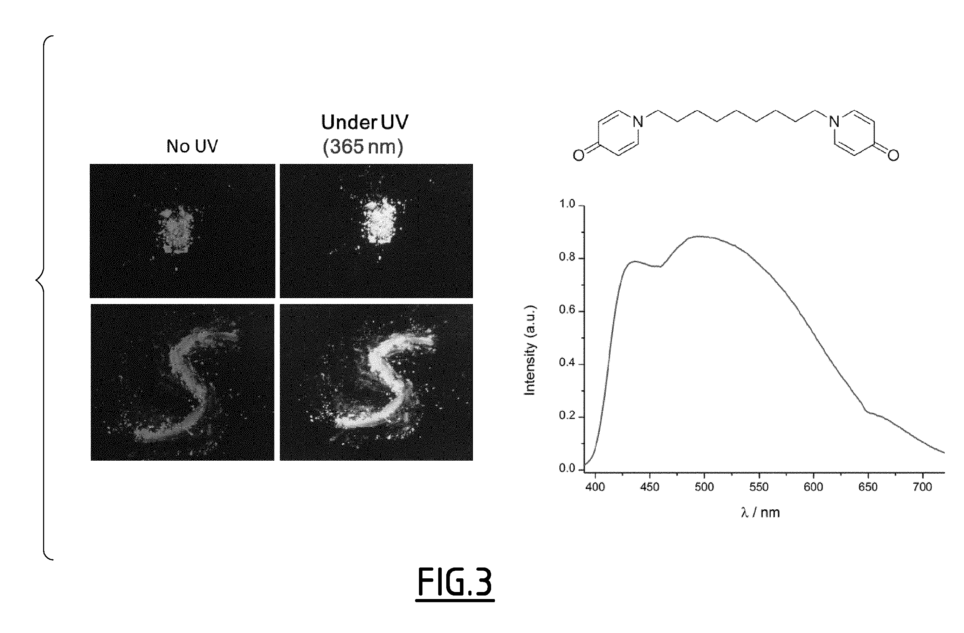

FIGURE 3. (Left) Photos of a powder of molecule **2** in absence of UV irradiation and under excitation at 365 nm. (Right) Solid state emission spectrum of **2** obtained as described, under excitation at 365 nm.
FIGURE 4. Molecule **2** emits white blue light in the solid state upon excitation at 365 nm, with CIE coordinates are (0.28, 0.35).

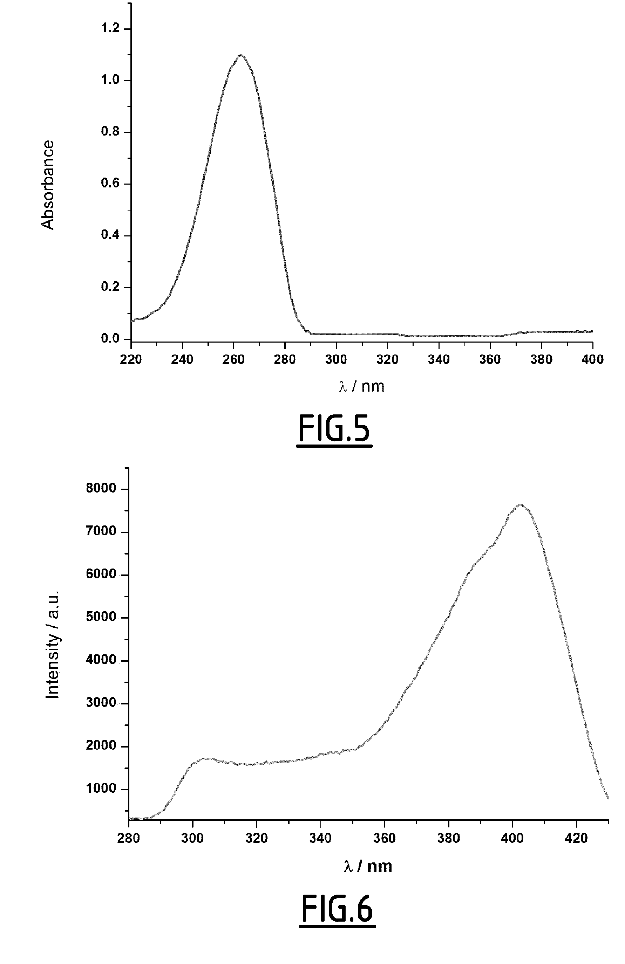

FIGURE 5. Absorption spectrum of a solution (1×10⁻⁵ M in methanol) of molecule **3.**
FIGURE 6. Solid state excitation spectrum of molecule **3** powder obtained as described.

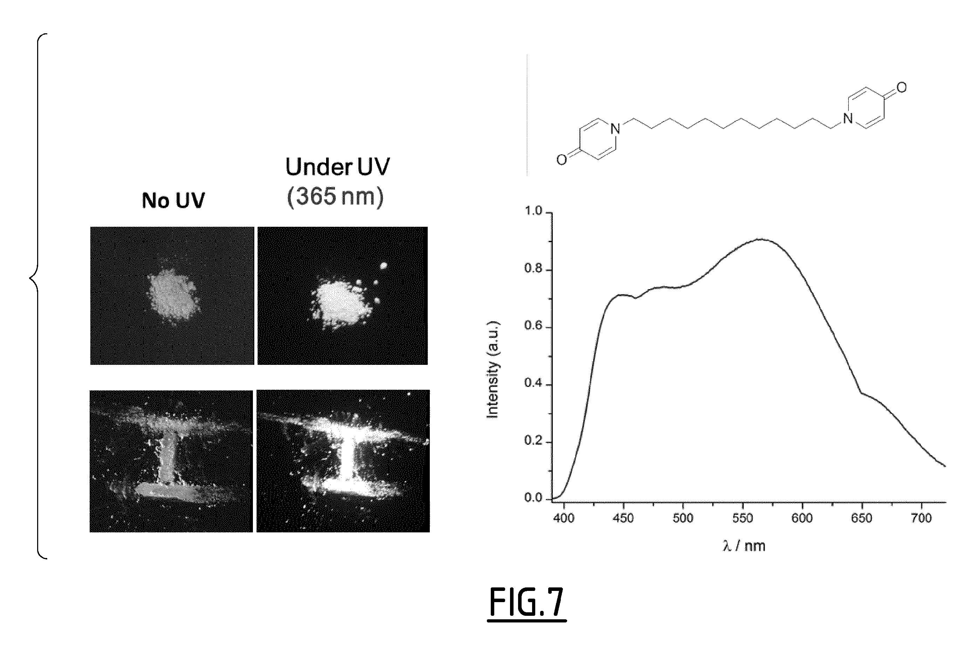

FIGURE 7. (Left) Photos of a powder of molecule **3** in absence of UV irradiation and under excitation at 365 nm. (Right) Solid state emission spectrum of **3** powder obtained as described, under excitation at λₑₓ = 365 nm. Single molecule **3** can emit almost pure white light. The same emission spectrum was obtained for powders of **3** by evaporation of solutions in water or in organic solvents (chloroform, ethanol, acetonitrile).

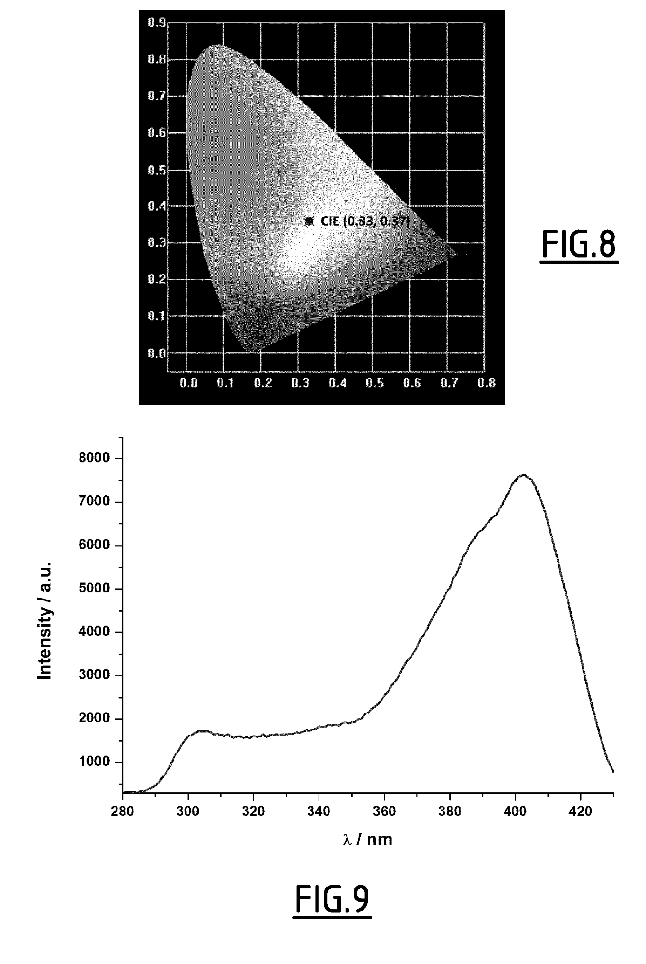

FIGURE 8. The CIE coordinates of molecule **3** are (0.33, 0.37).
FIGURE 9. Solid state excitation spectrum of molecule **4** powder obtained as described, under excitation at λₑₓ = 365 nm.

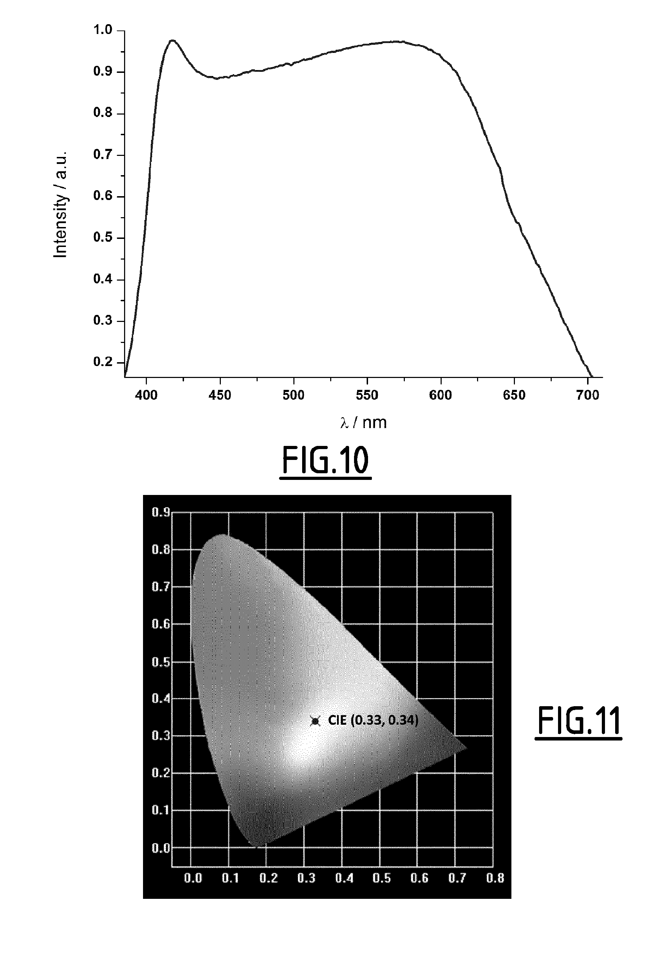

FIGURE 10. Solid state emission spectrum of molecule **4** powder obtained as described, λₑₓ = 365 nm.
FIGURE 11. CIE coordinates of molecule **4.** The single molecule emits pure white light upon 365 nm excitation.
FIGURE 12 CIE coordinates of points A (0.25, 0.22); B (0.33, 0.22); C (0.40, 0.38); D (0.35, 0.38); E (0.25, 0.30) defining a simple pentagon.

### Examples

The following examples illustrate the invention without any limitation. Starting materials are known or commercially available, or may be prepared by known methods. Percentages are expressed by weight, unless specified to the contrary.

### Example 1

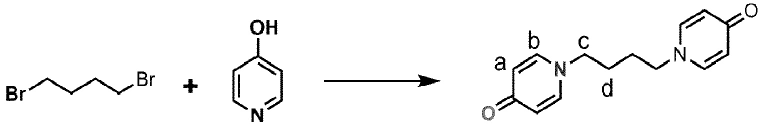

*1,1'-(butane-1,4-diyl)bis(pyridin-4(1H)-one)* (**1**): Before the reaction, 4-hydroxypyridine was recrystallized from chloroform and the solvent was distilled. Compound **1** was obtained by a similar procedure as for **3** except for using 1,4-dibromobutane instead of 1,12-dibromododecane. After the reaction was complete, the solvent was removed under vacuum to afford a yellow solid that was washed with acetone, and dried. The compound was obtained in 99.5 % purity (as judged by ¹H-NMR integration) as a yellowish solid. Yield: 72%. Melting point: 279.1°C. ¹H-NMR (400 MHz, DMSO-*d6*): δ 1.63 (s, 4H, H*d*), 3.86 (t, 4H, H*c*), 6.07 (d, 4H, H*b*), 7.64 (d, 4H, H*a*). ¹³C-NMR (100 MHz, DMSO-*d6*, 25 °C): δ = 177.6, 141.3, 117.6, 55.3, 27.3.

### Example 2

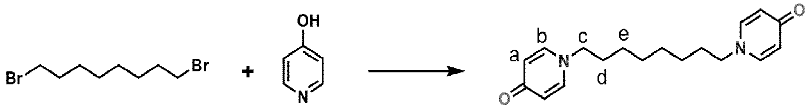

*1,1'-(octane-1,8-diyl)bis(pyridin-4(1H)-one)* **(2):** Before the reaction, 4-hydroxypyridine was recrystallized from chloroform and the solvent was distilled. Compound **2** was obtained by a similar procedure as for **3** except for using 1,8-dibromooctane instead of 1,12-dibromododecane. After the reaction was complete, the solution was filtered and the filtrate was evaporated under vacuum to give a yellow solid that was washed with acetone and recrystallized from a solution in hot acetonitrile on cooling. The target molecule was obtained in 99.9 % purity (as judged by ¹H-NMR integration) as a yellowish solid. Yield: 83%. Melting point: 90.5°C. ¹H-NMR (400 MHz, D₂O, 25 °C):δ 1.16 (s, 8H, H*e*), δ 1.69 (m, 4H, H*d*), 3.93 (t, 4H, H*c*), 6.49 (d, 4H, H*b*), 7.75 (d, 4H, H*a*). ¹³C-NMR (100 MHz, D₂O, 25 °C):δ = 220.8, 211.8, 205.3, 190.3, 183.3, 182.8, 182.1. MS (ESI+, H₂O, *m*/*z*): calcd for [M + H]⁺, 300.39; found, 300.81.

### Example 3

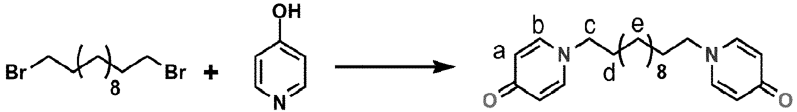

*1,1'-(dodecane-1,12-diyl)bis(pyridin-4(1H)-one)* **(3):** Before the reaction, 4-hydroxypyridine was recrystallized from chloroform and the solvent was distilled. A 100 ml three-necked round bottom flask equipped with a magnetic stirrer, additional condenser and thermometer were charged with 4-hydroxypyridine (1.2 g, 12 mmol), K₂CO₃ (2.1 g, 15 mmol), 1,12-dibromododecane (1.6 g, 5 mmol) and CH₃CN (30 ml). The reaction suspension was heated to reflux at 83°C for 24 hours. After the reaction was complete, the mixture was separated by hot filtration through a filter paper, and the filtrate was evaporated at 40°C under vacuum (45mmHg) to give the crude product as a light yellow solid. The residue on the filter was the carbonate salt and was discarded. ¹H NMR confirmed that the crude product contained three different compounds, unreacted 4-hydroxypyridine (6.3%), the desired symmetrical main product **(3)** of general type **Ib** (84%), and the unsymmetrical by-product of type **Ic** (9.7%). The crude product was washed by cold water three times to remove unreacted 4-hydroxypyridine and washed with acetone two times to remove the byproducts. After that, the symmetrical product (**Ib**) was recrystallized from a solution in hot acetonitrile on cooling. The purity was increased to 99.7% (as judged by ¹H-NMR integration). It was obtained as a pale yellow colored powdery solid and was dried overnight under vacuum. Yield: 76%. Melting point: 106.9 °C. ¹H-NMR (400 MHz, DMSO-d6, 25 °C):δ 1.24 (s, 16H, He), δ 1.66 (m, 4H, Hd), 3.83 (t, 4H, Hc), 6.07 (d, 4H, Hb), 7.67 (d, 4H, Ha). 13C-NMR (100 MHz, DMSO-d6, 25 °C):δ = 177.6, 141.3, 117.8, 55.7, 30.7, 29.3, 28.9, 25.9. MS (ESI+, MeOH, m/z): calcd for [M + H]+, 357.24; found, 357.31.

The crystal structure of the unsymmetrical isomer **3c** of compound **3** was determined. It confirmed the structure.

The lifetime measurement reveals a monoexponential decay feature with τ = 2.3 ns. Such lifetimes are characteristic of ligand-centered (LC) emissions, may originating from π-π* transition as well as intra-ligand charge-transfer (ILCT) due to the keto form of the molecule. The quantum yield of **3** was measured for 22% at room temperature.

### Example 4

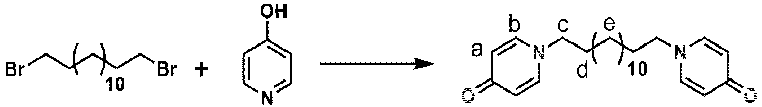

*1,1'-(tetradecane-1,14-diyl)bis(pyridin-4(1H)-one)* **(4)** was obtained as a light yellow powdery solid by a similar procedure as for **3** except for using 1,14-dibromotetradecane instead of 1,12-dibromododecane. The crude product contained three different compounds, unreacted 4-hydroxypyridine (6.5%), the desired symmetrical main product **4** of general type **Ib** (85.6%), and the unsymmetrical by-product of type **Ic** (7.9%). After the purification procedure as same as **3,** compound **4** was obtained in 99.2 % purity (as judged by ¹H-NMR integration). Yield: 65%. Melting point: 91.1°C. ¹H-NMR (400 MHz, CD₃OD-*d4*): δ 1.33 (s, 20H, He), δ 1.84 (m, 4H, H*d*), 4.01 (t, 4H, H*c*), 6.45 (d, 4H, H*b*), 7.86 (d, 4H, H*a*). ¹³C-NMR (100 MHz, DMSO-*d6*, 25 °C):δ = 177.4, 141.5, 117.3, 55.6, 30.7, 29.4, 28.9, 26.0. MS (ESI+, MeOH, *m*/*z*): calcd for [M + H]⁺, 385.27; found, 385.02.

The lifetime measurement reveals a monoexponential decay feature with τ = 2.5 ns. Such lifetimes are characteristic of ligand-centered (LC) emissions, may originating from π-π* transition as well as intra-ligand charge-transfer (ILCT) due to the keto form of the molecule. The quantum yield of **4** was measured for 23% at room temperature.

## Claims

1. Compound of formula (**I**) 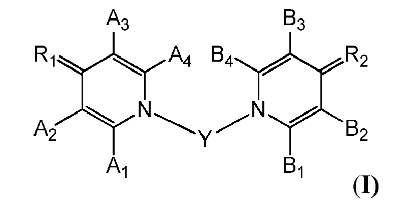 wherein Y represents a straight or branched alkyl chain having from 1 to 30 carbon atoms, preferably from 2 to 8 or from 9 to 20 carbon atoms, said alkyl chain optionally containing one or more unsaturations, and/or optionally containing one or more heteroatoms, and/or being optionally substituted, said alkyl chain being optionally interrupted by an aromatic group,
- R₁ and R₂ represent each independently an oxygen atom or 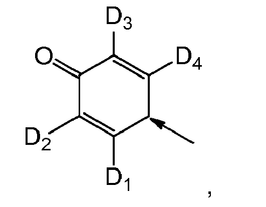
- ↗ indicating the point of attachment of the covalent double bond,
- A₁, A₂, A₃, A₄, B₁, B₂, B₃, B₄, D₁, D₂, D₃, and D₄ are identical or different, and represent each independently a hydrogen atom, a halogen atom (F, Cl, Br, or I), an alkyl, O-alkyl or aromatic group, A₁-A₂, A₃-A₄, B₁-B₂, B₃-B₄, D₁-D₂, and D₃-D₄ may each represent independently an aromatic ring.

2. Compound according to claim 1, wherein said alkyl chain or group is selected from the group consisting of methane, ethane, propane, butane, pentane, hexane, octane, nonane, decane, dodecane, tridecane, tetradecane, pentadecane, hexadecane, heptadecane, octadecane, nonadecane, eicosane, isopropane, tert-butyl, 2-ethylhexane, 2-methyl butane, 2-methylpentane, 1-methylpentane, 3-methylheptane, benzene, naphthalene, anthracene, perylene, diphenyl, bipyridine and terpyridine.

3. Compound according to claim 1 or 2, wherein said alkyl chain or group contains one or two heteroatoms, said heteroatom being independently selected from O, N, and S.

4. Compound according to any one of claims 1 to 3, wherein at least one carbon atom of said alkyl chain or group is substituted by one of the following atoms or groups:
- F, Cl, Br, or I,
- C₁-C₃₀ alkyl, C₃-C₇ cycloalkyl, C₆-C₁₀ aryl, heteroaryl, aralkyl, heteroarylalkyl, wherein said alkyl or aryl is optionally substituted by 1 to 3 groups R²⁰, wherein R²⁰ is one of the following:
- OR²², NR²³R²⁴, NHOH, NO₂, CN, CF₃, C₁-C₆ alkyl, C₂-C₆ alcenyl, C₂-C₆ alcynyl, C₆-C₁₀ aryl, aralkyl, =O, C(=O)R²², CO₂R²², OC(=O)R²², C(=O)NR²³R²⁴, OC(=O)NR²³R²⁴, NR²¹C(=S)R²² or S(O)_{y}R²² ;
- R²² is, at each occurrence, independently selected among H, C₁-C₃₀ alkyl, preferably C₅-C₂₀ alkyl, C₆-C₁₀ aryl and aralkyl ;
- R²³ and R²⁴ are, at each occurrence, independently selected among H, C₁-C₃₀ alkyl, preferably C₅-C₂₀ alkyl, C₆-C₁₀ aryl, aralkyl, or R²³ and R²⁴ form together a heterocyclic group having from 3 to 7 atoms.

5. Compound according to any one of claims 1 to 4, wherein said alkyl chain Y is interrupted by an aromatic group and is represented by: 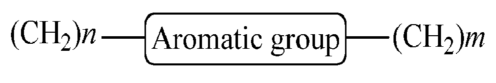 Wherein *n* and *m* are integers and wherein 2 < n + m < 30.

6. Compound according to any one of claims 1 to 5, wherein said aromatic group is a phenyl group, optionally substituted.

7. Compound according to any one of claims 1 to 6, wherein 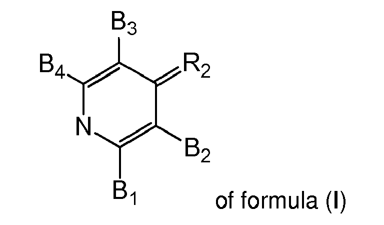 represents one of the following chemical groups: 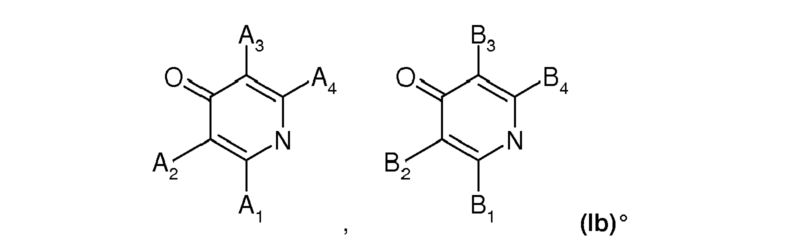 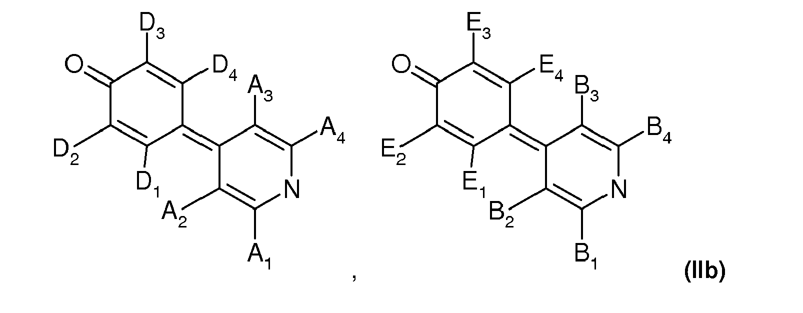 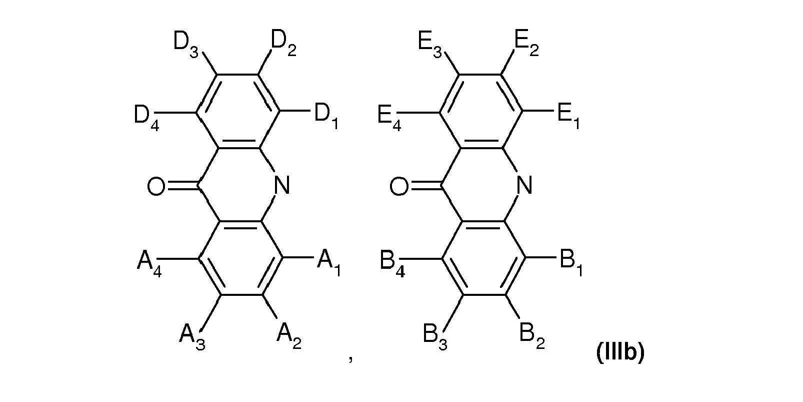 wherein A₁, A₂, A₃, A₄, B₁, B₂, B₃, B₄, D₁, D₂, D₃, and D₄ are defined according to any one of claims 1 to 5, and E₁, E₂, E₃, E₄ are identical or different, and represent each independently a hydrogen atom, a halogen atom (F, Cl, Br, or I), an alkyl, O-alkyl or aromatic group, E₁-E₂, and E₃-E₄ may each represent independently an aromatic ring.

8. Compound according to any one of claims 1 to 7, wherein said compound is represented by one of the following formulae:

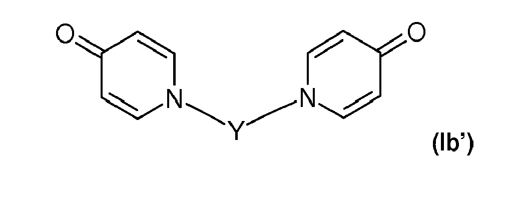

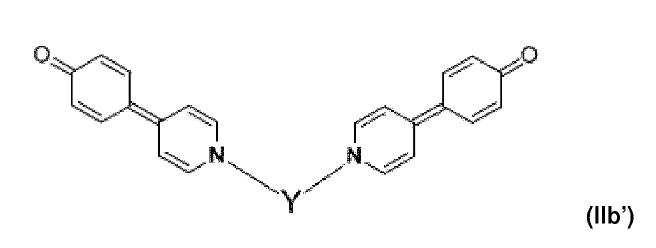

9. Compound according to any one of claims 1 to 8, wherein said compound is represented by the following formula: 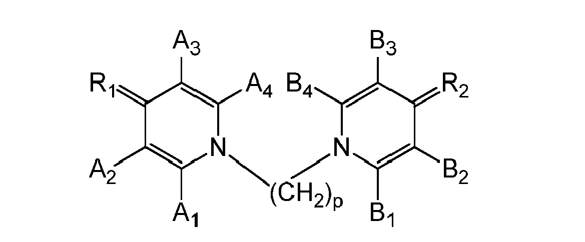 wherein *p* is an integer and wherein 1 < p < 30.

10. A solid material comprising or consisting of at least one compound according to any one of claims 1 to 9.

11. A light emiting device comprising at least one compound according to any one of claims 1 to 9, or of a solid phase according to claim 10.

12. Use of at least one compound according to any one of claims 1 to 9, or of a solid phase according to claim 10, for emiting white light.

13. A method for preparing a compound according to any one of claims 1 to 9, wherein said method comprises the following steps : 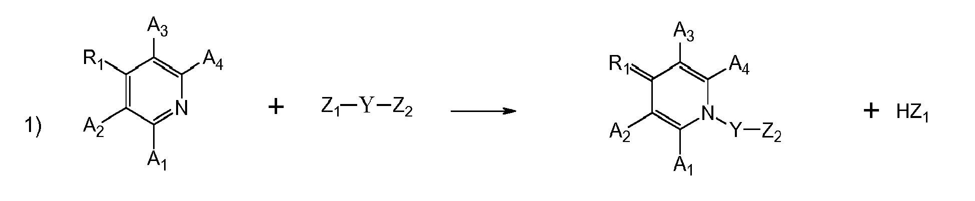 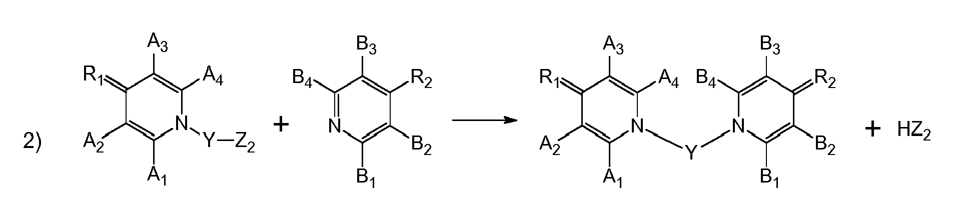 wherein Z₁ and Z₂ represent each independently leaving groups, preferably Z₁ and Z₂ represent each independently a halogen atom (preferably Br), a sulfonyl group, preferably a tosyl, a methanesulfonyl, a triflyl, a besyl, or a sulfonate group, or a sulphate ester group (-O-SO₃-R);
R₁, R₂, Y, A₁, A₂, A₃, A₄, B₁, B₂, B₃, and B₄ having the meaning according to any one of claims 1 to 9.
